(19) Europäisches Patentamt
European Patent Office
Office européen des brevets

(11) EP 2 716 329 A1

(12) EUROPEAN PATENT APPLICATION

(43) Date of publication:
**09.04.2014 Bulletin 2014/15**

(51) Int Cl.:
***A61Q 13/00*** *(2006.01)* ***C07H 15/04*** *(2006.01)*

(21) Application number: **13184656.0**

(22) Date of filing: **17.09.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **04.10.2012 TR 201211372**

(71) Applicant: **Viking Temizlik Ve Kozmetik Ürünleri Pazarlama Sanayi Ticaret Anomim Sirketi**
**Izmir (TR)**

(72) Inventors:
- **Hemen, Ceyda**
**35170 IZMIR (TR)**
- **Serin, Selahattin**
**01330 ADANA (TR)**
- **Avci, Hayrettin**
**35170 IZMIR (TR)**

(74) Representative: **Iskender, Ibrahim**
**Destek Patent, Inc.**
**Konak Mah. Lefkose Cad. NM Ofis Park**
**B Block No: 36/5**
**Besevler Nilüfer**
**16110 Bursa (TR)**

# (54) A fixer that increases the durability of essences and a method for obtaining said fixer

(57) The invention relates to ethyl glycoside fixer used for providing durability of essences and obtaining method for said fixer.

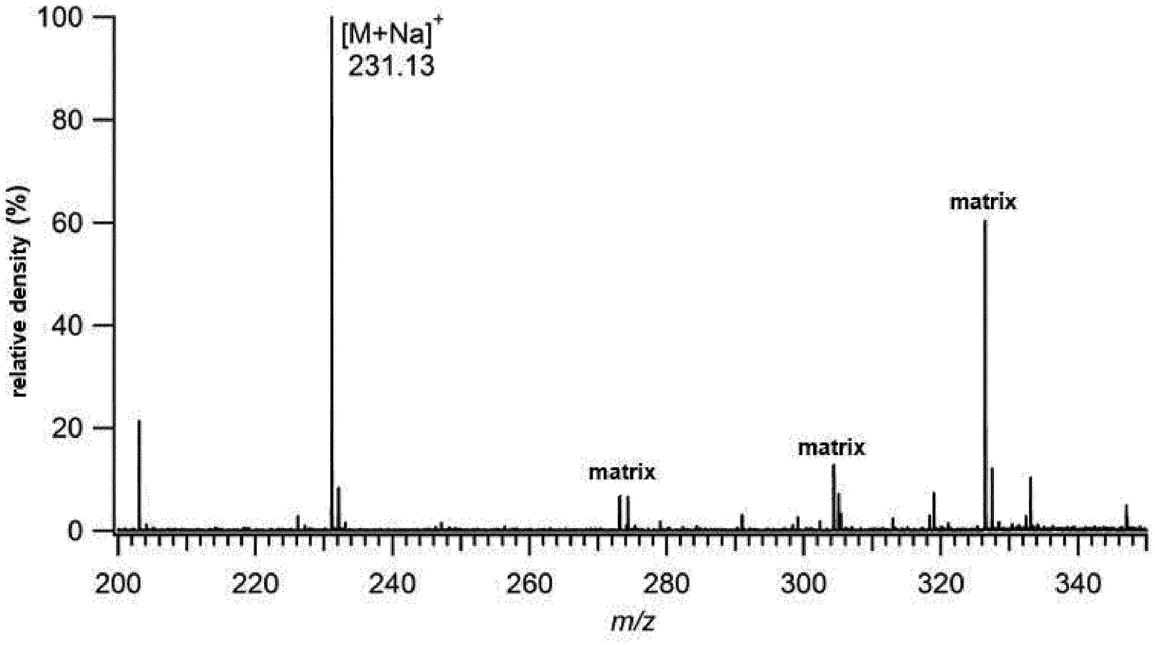


**Figure 1**

EP 2 716 329 A1

## Description

### Technical Field

The invention relates to ethyl glycoside that is used for increasing durability of essences and obtaining method for said ethyl glycoside.

The invention particularly relates to ethyl glycoside synthesis by using silica based heterogeneous acid catalyzer as catalyzer, and use of synthesis ethyl glycoside as a fixer in essences for increasing durability.

### State of Art

Essences are complex mixtures of synthetic aromatic chemicals and natural volatile oils, wherein they are widely used. In the highly competitive market of today, any daily used products are offered to consumers by attracting their attention by means of different aspects of the products. Particularly, the basic need materials such as cosmetics and cleaning materials that we find in every aspect of life are made to be more attractive with fragrance appealing to the emotions of the people. Gaining success in said products depends on the smell type and quality of the essences used.

The structure of essence is like a pyramid. Looking at the cross section of this pyramid, it is clear to see that an essence comprises of 3 main functional groups. Said groups are top note, middle note and foot note. Top note comprises volatile components, and is effective within the first 10 minutes. Middle note is midlevel volatile, and is effective within the first 15-20 minutes. Foot note is the part that provides the durability of essences, and comprises longest lasting and less volatile substances. Said part has a vital role on the economical success of a perfume.

Said essences are one of the most important factors for cosmetics and cleaning products in terms of creating and reflecting the aim of the product and suitable for the cooperate identity, and providing the permanency of this image in the eye of the consumers. The raw material that provides the durability of said essences are fixers. As a result of intermolecular interaction between scent components (ethereal, aldehyde, esteric...) of odorous oils and fixers; said fixers fix the evaporation ratio by regulating it, and provide that the scent components evaporate in a longer period of time. They do not change the odor type and chemical structure of the essence.

Within the researches in the literature, we come across some applications with respect to this subject:

> One of them is the patent application with the number US 7196052 B2 named as "Fragrance Composition". Said patent relates to essence diffusion and durability of glycerin ethers without changing the odor of the perfume in cosmetics, toilet-bathroom products and pharmacy.

One of them is the patent application with the number US 4324703 (A). In said patent, the fixer features of methyl glycoside poliols are analyzed.

Another one of them is the patent application with the number US 4683297 (A) titled as "Process for the synthesis of glycosides ". Within said invention, the corresponding glycoside is synthesized in the perfluorosulfonic acid resin catalyzer.

Another one of them is the patent application with the number US 2009165360 (A1). In said patent the fixative features of TPDM diesters are analyzed.

Another one of them is the patent application with the number US 4795738 (A). Said patent relates to perfume or scent compositions containing arbozol.

Another one of them is the patent application with the number US 4849400 (A). Said invention relates to durability characteristics of the fixer featured raw materials in the essences while using different amount and type of raw materials.

Another one of them is the patent application with the number US 5120709 (A). Within said patent; studies in order to increase scent quality using essence compositions and fixer are analyzed.

Another patent is the one with the publication number US 6147049. In said patent the fixative features of tetrahydronaphthalenes are analyzed.

It has been determined by analyzing IFRA (International Fragrance Association) standards that the fixer featured raw materials used in the essences in the art and in the above-mentioned patents are substantially allergen.

Consequently, it has been made necessary to make a development in the corresponding technical field in order to increase the durability of essences regarding the fixers that are non-allergen and unharmful for human health due to the abovementioned disadvantages and incapability of current solutions about this subject.

### The Object of the Invention

The present invention relates to a fixer that meets the above-mentioned requirements, removes all the disadvantages, brings some additional advantages and increases the durability of essences, and the method for obtaining said fixer.

The primary object of the invention relates to the use of ethyl glycoside as a fixer so as to increase the durability of essences.

One object of the invention is that the fixer ethyl glycoside both reduces the essence amount required in essences, and exhibit a better performance, with regard to current situation, even at this reduced essence proportion.

Another object of the invention is that said fixer does not harm human health, since the raw materials used in said ethyl glycoside synthesis are not allergen.

Another object of the invention is that in the synthesis of said ethyl glycoside, absolutely domestic resources are utilized. By this means, the essentiality for exogenous purchase goes away, and this helps the budget.

A similar object of the invention is to use silica based heterogeneous acid catalyzer in said fixer synthesis instead of the Nafion (perfluorosulphonic) catalyzer used currently in the art.

In order to carry out the abovementioned objects; the invention is a fixer to provide durability of essences, wherein said fixer is ethyl glycoside.

In order to carry out the objects of the invention, said ethyl glycoside contains silica based heterogeneous acid catalyzer.

In order to carry out the objects of the invention, said ethyl glycoside contains 15-25 % dextrosemonohydrate, 0.5-2.0% silica based heterogeneous acid catalyzer, 50-80% ethyl alcohol by weight.

In order to carry out the abovementioned objects; the invention is the method for ethyl glycoside fixer used for providing durability in essences, wherein it comprises the steps of;

- blending the dextrose monohydrate and ethyl alcohol along with the silica based heterogeneous acid catalyzer in a reaction container at 3-6 bar pressure, between 110 - 150 °C at 400 - 700 rpm about 1 - 3 hours, and
- depressurizing by cooling down the reaction following the reaction process.

All the structural and characteristic features and all the advantages of the invention will be more clearly understood from the following figures and detailed description for referring to these figure and for this reason, it is necessary that the evaluation be done by taking these figures and detailed description into account.

## Figures that Shall Assist in Understanding the Invention

**Figure 1** is the graphic showing raw materials used in the synthesis of ethyl glycoside, and its molecular weight.
**Figure 2** is the graphic showing a different catalyzer used in the prior art, and the molecular weight of the ethyl glycoside synthesis.
**Figure 3** is the graphic showing the exogenous raw materials used in the prior art, and the molecular weight of the ethyl glycoside synthesis.
**Figure 4** is the graphic in which 3 compared samples are evaluated with regard to their scent intensity.

The drawings do not need to be scaled necessarily and the details that are not necessary to figure out the present invention may have been ignored. Apart from this, the elements that are at least substantially identical and that have substantially identical functions are shown with same numbers.

## Detailed Description of the Invention

Within this detailed description, a fixer that increases durability in essences according to the invention, and preferred embodiments of the method for obtaining said fixer are disclosed just for providing that the subject can be better understood and without any limiting effect.

The invention relates to ethyl glycoside synthesis by using silica based heterogeneous acid catalyzer as catalyzer, and use of synthesis ethyl glycoside as a fixer in essences for increasing durability.

The fixer according to the invention is ethyl glycoside. The features of said ethyl glycoside that the materials used in the synthesis of it do not harm human health, since they do not have allergen characteristics. Another feature of the invention is that domestic sources are utilized in production of said raw materials. Thanks to this, the external dependence disappears.

*Said ethyl glycoside is synthesized, as given in the following reaction, along with the dextrose monohydrate, ethyl alcohol and a catalyzer:*

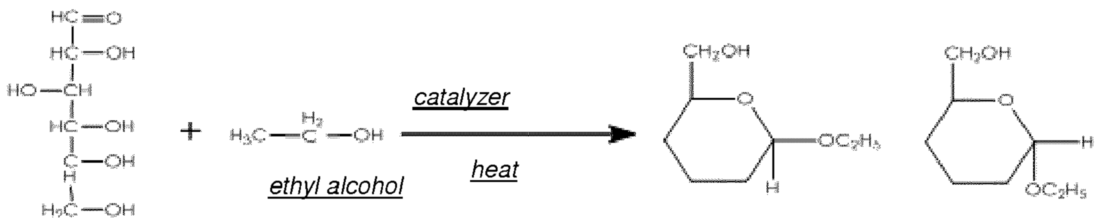


*ethyl alcohol*

*dextrosemonohydrate* *α-ethyl glycoside* *β-ethyl glycoside*

Another feature of said invention is that it uses silica based heterogeneous acid as catalyzer in said ethyl glycoside synthesis. This catalyzer comprises many advantages in comparison with the Nafion (poly(perfluorosulphonic acid)) catalyzer that is used in the current art.

The Nafion used in the art comprises a gelatin-like structure. As a result of which, it disintegrates as it decomposes following the reaction. This circumstance causes difficulty in production. Another negative aspect of said Nafion is that it is expensive. Another disadvantage is that it is difficult to reuse due to the complication of percolation.

Yet, the newly used silica based heterogeneous acid catalyzer in ethyl glycoside synthesis comprises a solid chemistry. As a result of which, it decomposes easily following the reaction. This makes said production both easier and faster. Another superiority over the current art is that it is cheap. This helps the budget positively. One other and important difference is that it can be reused following reaction by separating from the reaction environment through filtration.

In said reaction; 1 - 10 gr dextrose monohydrate 0.01 - 1.0 gr silica based heterogeneous acid catalyzer and 5 - 25 gr ethyl alcohol is used. In this usage; said raw materials were put in reaction container. The reaction was carried out in a closed container at 3-6 bar pressure, between 110 - 150 °C at 400 - 700 rpm about 1 - 3 hours. Following the reaction process, the reaction was cooled and depressurizing happened. Positive results were obtained by performing tests on said synthesis process. In order to compare these said positive results, tests were conducted in laboratories regarding said synthesis method without constant pressure and by elongating the reaction duration at lower temperatures. Within said tests, it was figured out that the materials that were not reacting stayed in alcohol without dissolving, and this synthesis was not successful.

The raw materials in said reaction are as follows by their weight ratio:

| **Raw material** | **By weight %** |
|---|---|
| Dextrose monohydrate | 15-25% |
| Silica based heterogeneous acid catalyzer | 0.5-2.0% |
| Ethyl alcohol | 50-80% |

When the efficiency of said reaction is calculated, it is seen that it is 95.64%.

| Dextrose monohydrate | + | Ethanol | → Ethyl glycoside | +water |
|---|---|---|---|---|
| $M_A$:180.19 g/mol | | $M_A$:46.06844 g/mol | $M_A$:208.228 g/mol | |
| 1 mol | | 1 mol + more (as solvent) | 1 mol | |

The efficiency following the evaporation of the alcohol remaining in the reaction is 95.64%. The ethyl glycoside synthesis according to the invention is shown via graphics in Figure 1, 2 and 3 comparatively by getting synthesized with different catalyzer or exogenous raw materials.

In said Figure 1, the graphic is given that shows the molecular weight of ethyl glycoside is 231.13, which is obtained as a result of 1 - 3 hours of reaction time that includes 15-25% dextrose monohydrate (domestic), 0.5-2.0%

silica based heterogeneous acid catalyzer (according to the invention), and 50-80% ethyl alcohol (technical raw material-domestic).

In said Figure 2, the graphic is given that shows the molecular weight of ethyl glycoside is 231.13, which is obtained as a result of 1 - 3 hours of reaction time that includes 15-25% dextrose monohydrate (according to the invention), 0.5-2.0% Nafion (poly(perfluorosulphonic)) (old), and 50-80% ethyl alcohol (technical raw material-domestic).

In said Figure 3, the graphic is given that shows the molecular weight of ethyl glycoside is 231.13, which is obtained as a result of 1 - 3 hours of reaction time that includes 15-25% glycoside anhydride (exogenous), 0.5-2.0% Nafion (poly(perfluorosulphonic)) (old), and 50-80% ethyl alcohol (technical raw material-domestic).

As a result of corresponding graphics, it is seen that ethyl glycoside ($C_8H_{16}O_6$) with same molecular weight is synthesized. Accordingly, the synthesis of Figure 1 according to the invention has both the same molecular weight and advantages thanks to the domestic raw materials in it, and the catalyzer that has superiority over the catalyzer used in the current art.

Another evaluation of the ethyl glycoside that is used as fixer according to the invention is performed via the Glucam P20 (methylglycoside polyol) used as fixer in the current art. These conducted tests are for performance measurements of said fixers. These measurements have been applied to 3 samples.

Sample #1: 1.0 Essence

Sample #2: 0.9 Essence + 0.5% Glucam P20 (methylglycoside polyol)

Sample #3: 0.9% Essence + 0.5% Ethylglycoside (synthesized according to the invention)

| | 1 | 2 | 3 |
|---|---|---|---|
| **FIRST PRODUCT** | | | |
| Density | 5.75 | 5.13 | 6.38 |
| Coolness | 5.38 | 5.13 | 5.38 |
| **WET CLOTHES** | | | |
| Density | 5.38 | 4.63 | 5.25 |
| Coolness | 5.50 | 5.00 | 5.63 |
| **DRY CLOTHES** | | | |
| Density | 4.75 | 2.50 | 5.00 |
| Coolness | 4.75 | 2.75 | 5.00 |

According to the results of the tests, there comes the conclusion that the performance of the product with ethylglycoside particularly on dry clothes is twice as much comparing to the other two products. Said results are given in Figure 4 comparatively as density graphic.

In another evaluation of the fixer according to the invention has been carried out on the stability test operation of the 3 samples given above. During the monthly tests, it has been observed that the essence stability is agreeable as a result of view and scent control. At the end of the 6th month, the reactive indexes and solid substances of said samples have been checked. As they are same as said values, it means that the use of ethyl glycoside according to the invention does not cause decomposition in the quality of the essence.

| SAMPLES | Reactive Index (nD) | Solid Substance (%) |
|---|---|---|
| Essence + Ethyl Glycoside | 1.495 | 81.3 |
| Essence | 1.495 | 81.3 |
| Essence + Glucam P20 (Methyl Glycoside) | 1.495 | 91.3 |

Furthermore, m/z ratio is stated on the graphics in said figures. This ratio means mass to charge ration.

**In addition to** this, the trace amount of sodium and potassium in the solutions that are used while synthesizing any substance can be easily determined in mass spectrometer. Mass spectrometer is a precision device. Said alkali metals can smoothly hold onto the substance whose weight should be determined, and form additives (adduct). By this means, it is ordinary to see [M+Na]+ and [M+K]+ peaks on mass spectrum. In order to validate the study, 10 nM NaCl solution has been prepared into the substance to be analyzed and 1 microliter has been added. In this way, it is shown that the [M+Na]+ peak increases.

Moreover, organic molecules so-called as matrix are used for transferring the substances to gas phase, which are to be analyzed in MALDI (Matrix-assisted laser desorption/ionization) mass spectrometer. The principle of MALDI is the absorption of applied laser power by organic molecule and transfer of some of this power to the sample. During this transfer, the sample obtains proton in this ambiance and is converted into ion. The matrix used is 2-5 dihydroxybenzoic acid.

## Claims

1. A fixer to provide durability of essences, **characterized in that** said fixer is ethyl glycoside.

2. A fixer according to Claim 1, **characterized in that** it comprises silica based heterogeneous acid catalyzer.

3. A fixer according to Claims 1 - 2, **characterized in that** it comprises 15-25 % dextrosemonohydrate, 0.5-2.0% silica based heterogeneous acid catalyzer, 50-80% ethyl alcohol by weight.

4. A method for ethyl glycoside fixer used for providing durability in essences, **characterized in that** it comprises the steps of;

    - blending the dextrosemonohydrate and ethyl alcohol along with the silica based heterogeneous acid catalyzer in a reaction container at 3-6 bar pressure, between 110 - 150 °C at 400 - 700 rpm about 1 - 3 hours, and
    - depressurizing by cooling down the reaction following the reaction process.

5. Fixer obtaining method according to Claim 4, **characterized in that** it comprises 15-25% dextrose monohydrate, 0.5-2.0% silica based heterogeneous acid catalyzer, 50-80% ethyl alcohol by weight.

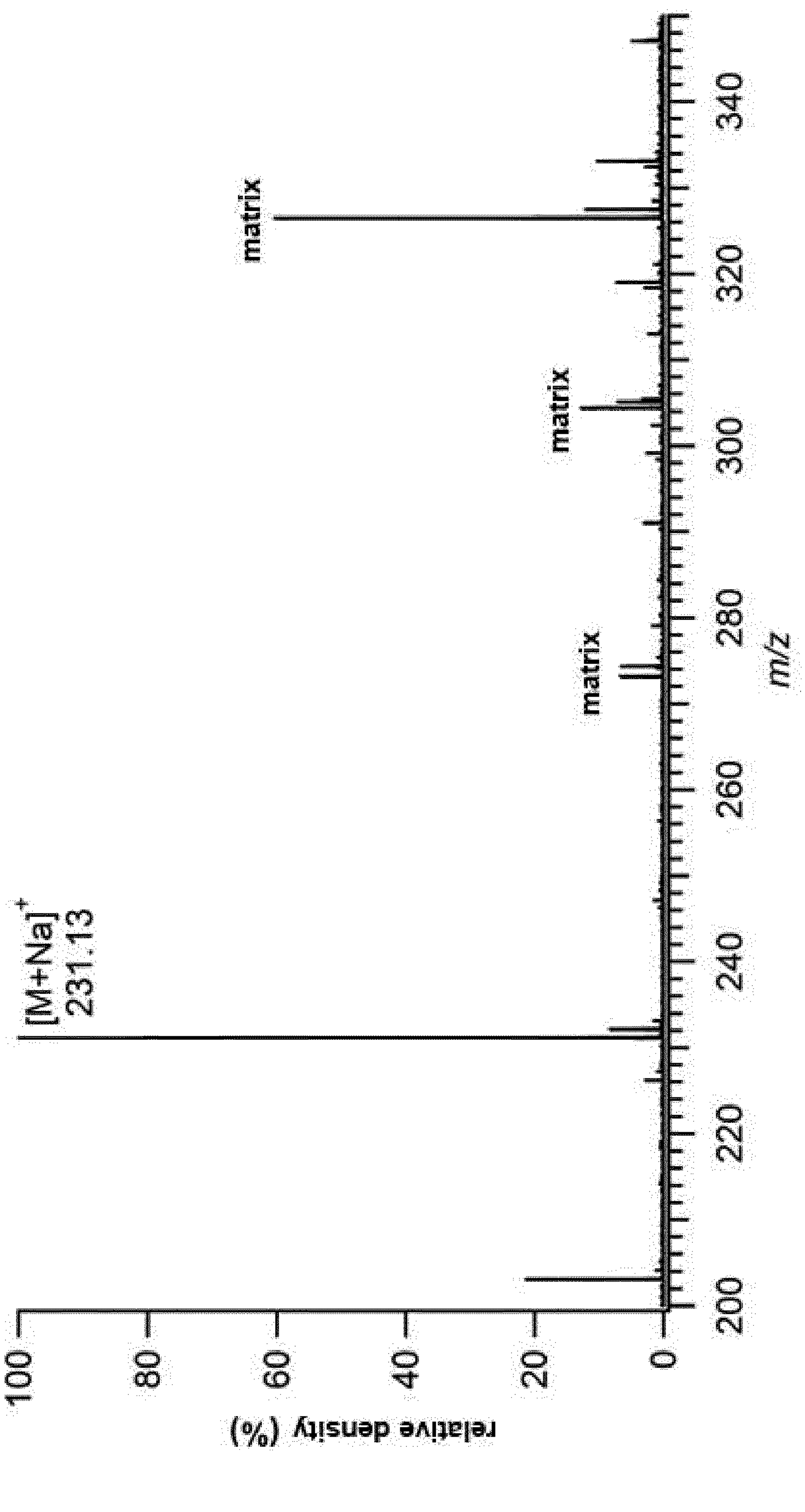
[M+Na]+
231.13
matrix
matrix
matrix
relative density (%)
100
80
60
40
20
0
200
220
240
260
280
300
320
340
m/z

Figure 1

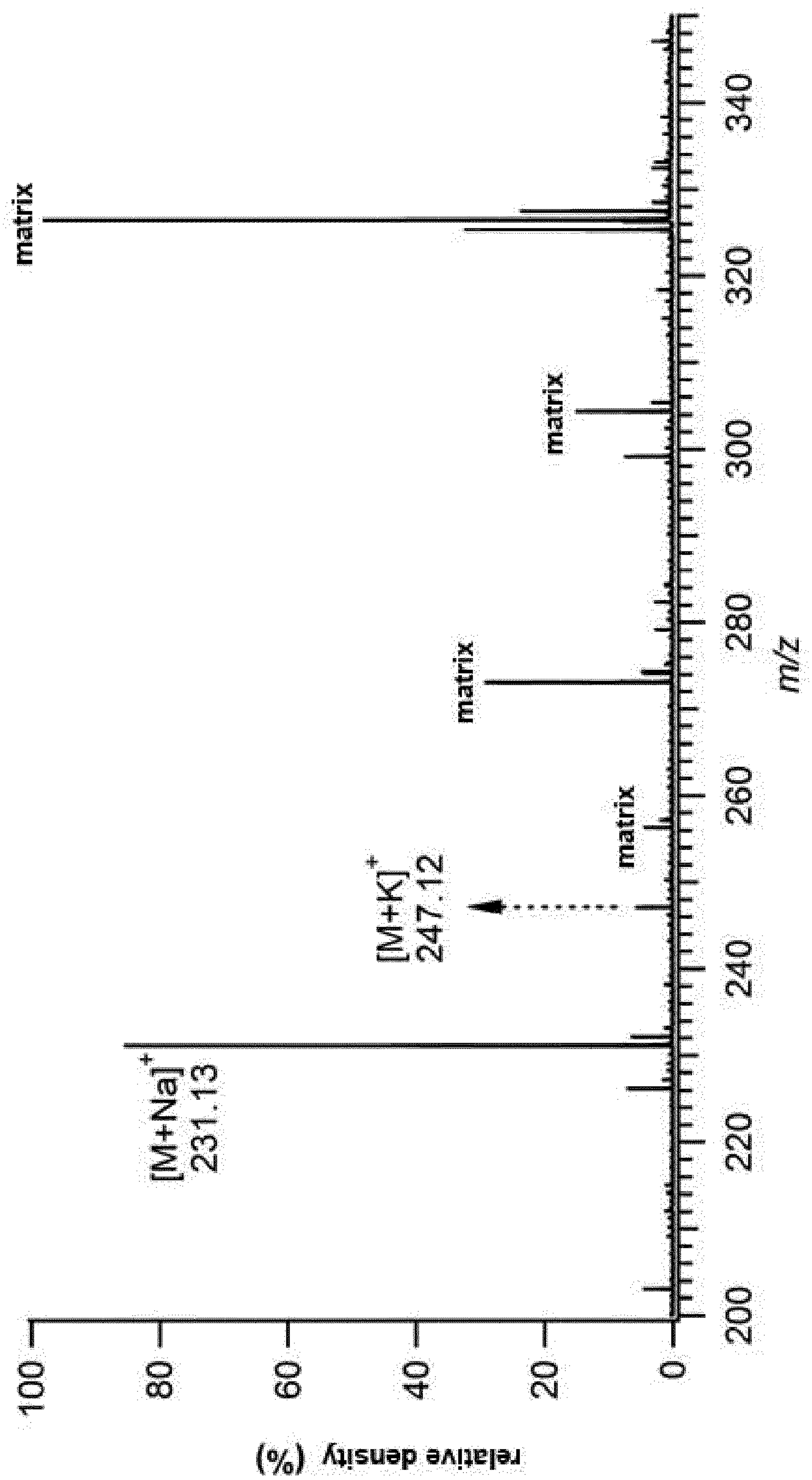

relative density (%)
100
80
60
40
20
0
200
220
240
260
280
300
320
340
m/z
[M+Na]+
231.13
[M+K]+
247.12
matrix
matrix
matrix
matrix


Figure 2

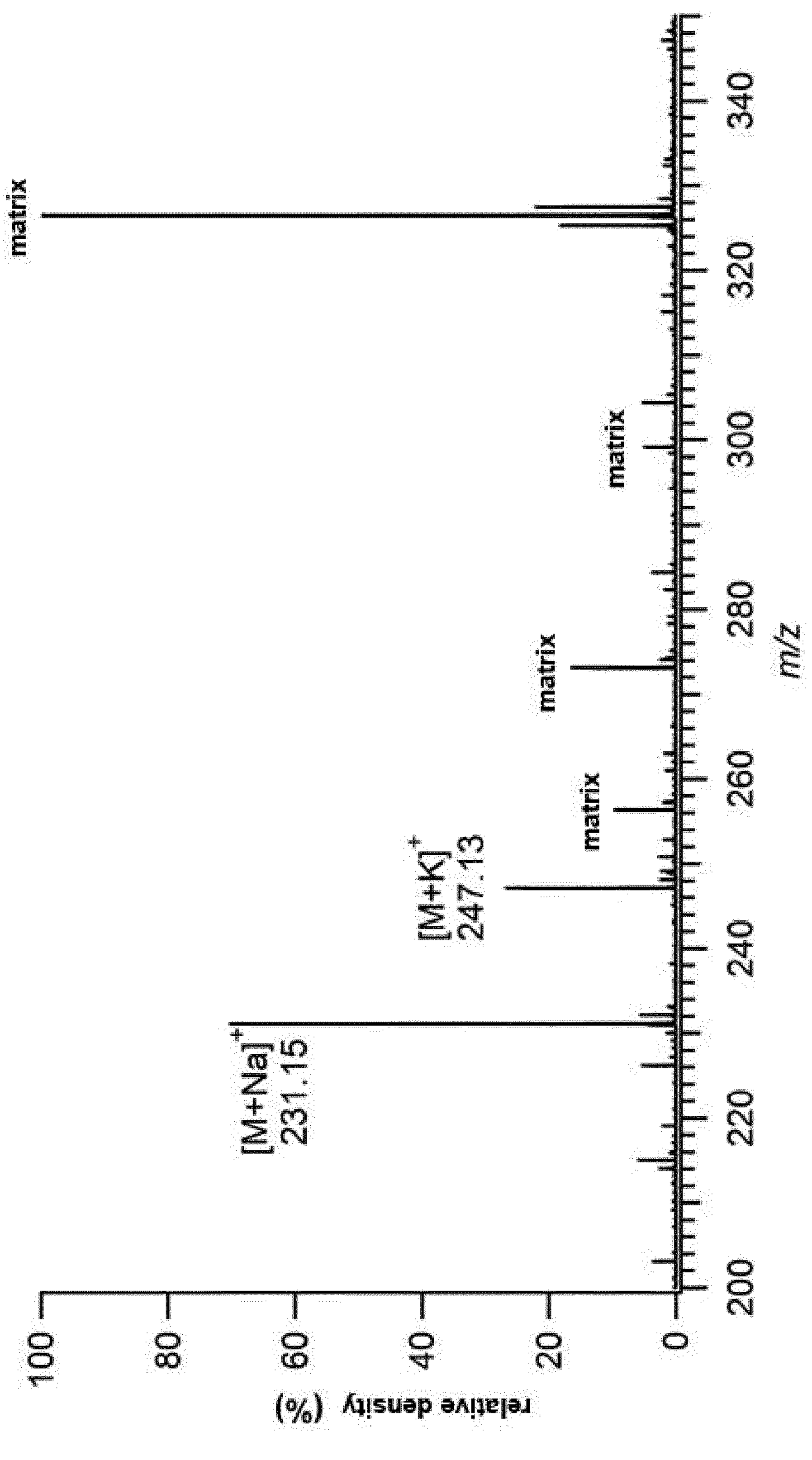

matrix
matrix
matrix
matrix
[M+Na]+
231.15
[M+K]+
247.13
100
80
60
40
20
0
relative density (%)
200
220
240
260
280
300
320
340
m/z


Figure 3

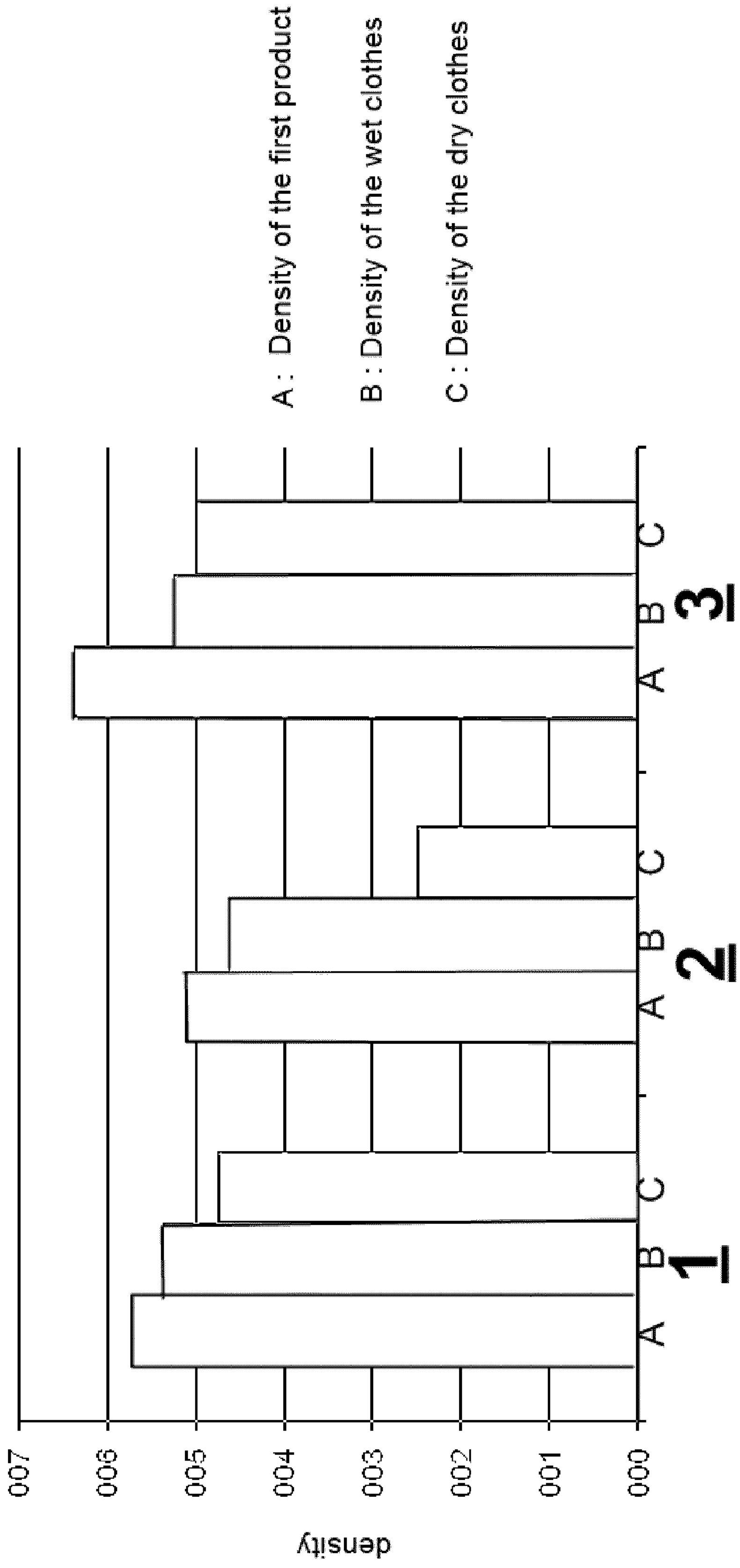
A : Density of the first product
B : Density of the wet clothes
C : Density of the dry clothes
density
007
006
005
004
003
002
001
000
A
B
C
1
A
B
C
2
A
B
C
3

Figure 4

Europäisches Patentamt
European Patent Office
Office européen des brevets

# EUROPEAN SEARCH REPORT

Application Number
EP 13 18 4656

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | US 4 324 703 A (SELDNER ABRAHAM) 13 April 1982 (1982-04-13) * examples 1-4 * ----- | 1-5 | INV. A61Q13/00 C07H15/04 |
| A | JP S62 84010 A (SHISEIDO CO LTD) 17 April 1987 (1987-04-17) * abstract * ----- | 1-5 | |
| A,D | US 5 120 709 A (CELLA JOHN A [US] ET AL) 9 June 1992 (1992-06-09) * example 1 * ----- | 1-5 | |
| A,D | US 2009/165360 A1 (DOBBS SUZANNE WINEGAR [US] ET AL) 2 July 2009 (2009-07-02) * example 1 * ----- | 1-5 | |
| A,D | US 4 849 400 A (KING JERRY [US]) 18 July 1989 (1989-07-18) * examples 3-6 * ----- | 1-5 | |
| X,D | US 4 683 297 A (YANAMI TETSUJI [JP] ET AL) 28 July 1987 (1987-07-28) | 1 | TECHNICAL FIELDS SEARCHED (IPC) |
| A | * example 6 * ----- | 2-5 | A61Q C07H |

The present search report has been drawn up for all claims

| Place of search | Date of completion of the search | Examiner |
|---|---|---|
| Munich | 6 February 2014 | Merkl, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 13 18 4656

This annex lists the patent family members relating to the patent documents cited in the above-mentioned European search report.
The members are as contained in the European Patent Office EDP file on
The European Patent Office is in no way liable for these particulars which are merely given for the purpose of information.

06-02-2014

| Patent document cited in search report | Publication date | Patent family member(s) | Publication date |
|---|---|---|---|
| US 4324703 A | 13-04-1982 | NONE | |
| JP S6284010 A | 17-04-1987 | NONE | |
| US 5120709 A | 09-06-1992 | NONE | |
| US 2009165360 A1 | 02-07-2009 | US 2009165360 A1 | 02-07-2009 |
| | | WO 2009085103 A2 | 09-07-2009 |
| US 4849400 A | 18-07-1989 | EP 0358298 A1 | 14-03-1990 |
| | | JP H0255797 A | 26-02-1990 |
| | | US 4849400 A | 18-07-1989 |
| US 4683297 A | 28-07-1987 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7196052 B2
- US 4324703 A
- US 4683297 A
- US 2009165360 A1
- US 4795738 A
- US 4849400 A
- US 5120709 A
- US 6147049 A